# EUROPEAN PATENT SPECIFICATION

(11) **EP 3 190 924 B1**
(45) Date of publication and mention of the grant of the patent: **26.02.2020**
(21) Application number: 15840125.7
(22) Date of filing: 10.09.2015
(51) Int. Cl.: A47C 7/74, A61G 7/00

(54) **A CONTROLLED CLIMATE BED WITH SLEEPER FEEDBACK**
BETT MIT KONTROLLIERTEM KLIMA UND SCHLÄFERRÜCKMELDUNG
LIT À ATMOSPHÈRE RÉGULÉE AVEC RÉTROACTION DE L'UTILISATEUR

(30) Priority: 10.09.2014 US 201462048528 P
(43) Date of publication of application: 19.07.2017
(73) Proprietor: Board of Regents, The University of Texas System, Austin, TX 78701 (US); Gentherm Incorporated, Northville, MI 48167 (US)
(72) Inventor: DILLER, Kenneth R., Elgin, Texas 78621 (US); BRYKALSKI, Michael Julius, South Lyon, Michigan 48178 (US); MARQUETTE, David Brian, Farmington Hills, Michigan 48334 (US)
(74) Representative: Icely, Dominic Michael
(86) International application number: PCT/US2015/049483
(87) International publication number: WO 2016/040676

(56) References cited:
- EP-A1- 2 320 292
- JP-A- 2008 119 454
- US-A- 5 448 788
- US-A1- 2002 124 312
- US-A1- 2011 041 246
- US-A1- 2011 314 837
- US-A1- 2012 227 182
- US-B1- 6 256 818

## Description

### PRIORITY

This application claims priority to U.S. Application No. 62/048,528, filed September 10, 2014.

### BACKGROUND

An important aspect of human comfort during sleep is thermal comfort. Empirical tools have been developed for determining air temperature and humidity that produce thermal comfort as a function of the level of physical activity, or metabolic rate, and insulating properties of the clothing that the person is wearing. Other research has documented that the human thermoregulatory system operates on a circadian cycle, much as does sleep, and that the thermoregulatory system is functionally involved in driving the sleep onset, continuation, and completion process.

For example, throughout the day a typical person will experience a relatively high body core temperature which peaks in the evening. As the body core temperature begins to drop, sleep onset occurs. The body core temperature continues to drop through the night. Prior to awakening in the morning, the process reverses and temperatures begin to rise, facilitating the completion of sleep. Much of the heat transfer that causes body core temperature changes occurs through glabrous skin, which in humans is skin that is naturally hairless, such as the skin found on the ventral portion of the fingers and toes, palmar surfaces of the hands, soles of feet, and other areas.

Modulation of blood flow to arteriovenous anastomoses ("AVAs") in glabrous skin of the hands and feet-commonly referred to as "distal blood flow"-plays a major role in determining whether quality sleep occurs. For example, vasodilated AVAs, which are associated with warm feet, have been shown to promote rapid onset of sleep, whereas vasoconstricted AVAs produce delayed sleep onset. The AVAs in glabrous skin function as the primary heat transfer portals between the body core and the environment.

Recently, bed and mattress manufacturers have become increasingly aware of the effects of thermoregulation on sleep quality. For example, memory foam mattresses are well known to be highly insulating, resulting in sleepers becoming overheated part way through the sleep cycle and causing sleep difficulty associated with a higher body core temperature thereafter. This drawback has been difficult to overcome, with some manufacturers relying on passive solutions such as embedding phase-change pellets in the upper layers of the mattress to store body heat by melting rather than an increase in substrate temperature.

The current methods of thermal regulation have particular drawbacks. For example, influencing thermal regulation by way of air temperature is highly inefficient and generally ineffective. Passive solutions, such as the phase-change pellets described above, have a limited capacity for heat absorption before becoming useless to temperature regulation. In addition, none of these methods take into account the changing temperature of the sleeper at different points in time during the sleep cycle. As a result, even if an ideal temperature can be achieved at one point during the sleep cycle, the sleeper's temperature may change to such an extent that the sleeper is unable to continue sleeping. Current systems and methods are unable to adapt to a sleeper in this type of situation. Additionally, current systems are not able to vary temperature based on, for example, the circadian temperature cycle that occurs during sleep. US2011/041246 A1 shows a climate-controlled bed which is similar to the one of the invention.

As a result, there is a distinct need for a system and/or method capable of maintaining a climate-controlled sleeping environment for a user. In particular, there is a need for a system and/or method that can monitor the temperature of a sleeper and react accordingly in a manner most beneficial for sleep. In addition, there is a need for an energy-efficient way to maintain a climate-controlled sleeping environment in order to reduce energy usage. The integration of closed-loop physiological feedback from a sleeper to create a personalized local thermal environment that will match the sleep comfort needs of an individual marks a major advance in the field of sleep studies.

Other systems, methods, features and/or advantages will be or may become apparent to one with skill in the art upon examination of the following drawings and detailed description. It is intended that all such additional systems, methods, features and/or advantages be included within this description and be protected by the accompanying claims.

### BRIEF DESCRIPTION OF THE DRAWINGS

The following detailed description will be better understood when read in conjunction with the appended drawings, in which there is shown one or more of the multiple embodiments of the present invention. It should be understood, however, that the various embodiments of the present invention are not limited to the precise arrangements and instrumentalities shown in the drawings.
Fig. 1 is an example embodiment of a climate-controlled bed having multiple climate zones.
Fig. 2 is an example embodiment of a sleeper in a sleeping position.
Fig. 3 is an example embodiment of a control system.
Fig. 4 is an example embodiment of a thermoelectric energy source providing cooling and/or warming air.

### DETAILED DESCRIPTION

A climate-controlled bed capable of adapting to the needs of a sleeper is provided. The bed includes a thermoelectric energy source, a sensor configured to monitor a physiological temperature of a sleeper, and a control system that regulates a temperature of the bed via the thermoelectric energy source. The control system can utilize data from the sensor to determine optimal thermal needs of the sleeper. The control system can also vary the temperature of the bed during a sleep cycle based on at least one predetermined sleep factor, such as the natural circadian temperature cycle.

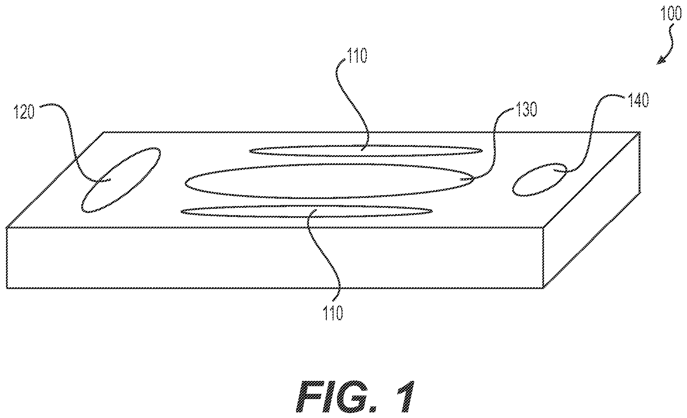

Fig. 1 shows an example embodiment of a climate-controlled bed. Bed 100 is shown as having a plurality of climate zones. For example, side climate zones 110 are located along the sides of bed 100. Foot climate zone 120 is located toward the foot end of bed 100. Central climate zone 130 is located in the center of bed 100. Finally, cervical-spine climate zone 140 is located near the head of the bed. In one example embodiment, side climate zones 110, foot climate zone 120, and cervical-spine climate zone 140 are used as warming zones. In this example embodiment, central climate zone 130 is used as a cooling zone.

In an example embodiment, a zone may be warmed or cooled via a thermoelectric energy source (not shown) to provide warming or cooling to these zones. Warming or cooling may be provided in any number of ways, including for example via air flow, other types of fluid flow, or electrical resistance. The thermoelectric energy source may be used to simultaneously provide warming and cooling capacity to different zones. According to the invention however the first thermal energy source is capable of simultaneously providing cooling and warming to separate air streams directed to different areas of the bed sleeping surface. Alternatively and not according to the invention, a plurality of thermoelectric energy sources may be implemented in order to have a dedicated energy source for warming and cooling, respectively.

In the example above, side climate zones 110, foot climate zone 120, and cervical-spine climate zone 140 are designated as warming zones, while central zone 130 is designated as a cooling zone. This example setup may be useful for increasing blood flow throughout the body by, for example, warming the extremities of the body as well as the cervical spine to promote blood flow. The increased blood flow would facilitate cooling from the central zone 130, as increased blood flow would lead to an increase in heat transfer from the sleeper to the bed.

In other example embodiments, more zones or fewer zones may be utilized by bed 100. Additionally, each zone may be designated as a cooling or warming zone. In some embodiments, the temperature of each zone may be varied according to the needs of the user. For example, if the user is exceedingly cool when first getting onto bed 100, all of the zones may be turned to their warming functions. As the user's temperature rises, the temperatures of the zones may be adjusted accordingly.

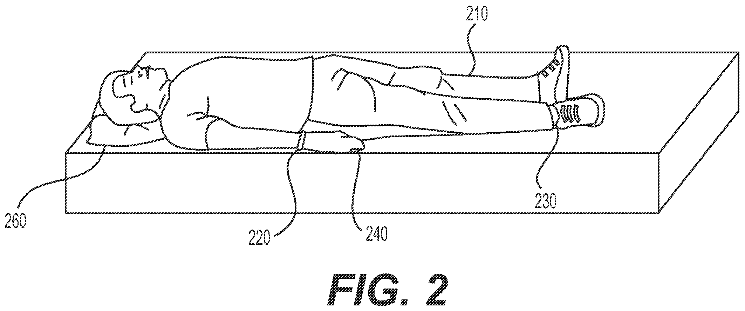

Monitoring the user may be done in a variety of ways. Fig. 2 shows an example embodiment of a user 210 in a sleeping position. The user is depicted as being fully clothed, but of course user 210 is likely to be dressed in appropriate sleep attire. In an example embodiment, user 210 is wearing a first sensor 220. First sensor 220 is shown as being attached to the arm of user 210, however the sensor may be placed in any other suitable location. Sensor 220 may measure a variety of physiological properties, including for example temperature, heart rate, blood pressure, and motion. Sensor 220 may be incorporated into a device such as a wrist band, forearm band, or the like.

Fig 2. discloses additional sensors as well. For example, a leg sensor 230 is shown attached to the user near the ankle. Leg sensor 230 may be placed on the calf or ankle such that it contacts non-glabrous skin and provides a temperature measurement for that type of skin. An additional sensor may be located on glabrous skin in order to provide a temperature comparison between the user's glabrous and non-glabrous skin, which may be interpreted as an indication of the level of blood flow to glabrous skin and of the status of the thermoregulatory function, especially in relation to the circadian cycle. For example, glabrous sensor 240 may be placed on the hands or feet to measure the temperature of the glabrous skin at those locations. Additionally, a finger clip pulse oximeter may be incorporated into, for example, sensor 240 for use on the user's finger. Additional sensors may be incorporated at other locations of the user's body.

Fig. 2 also depicts a pillow 260. In some embodiments, pillow 260 can function as an energy source providing either warming or cooling energy. Pillow 260, while shown in the form of a pillow, may also take the form of a heating/cooling blanket or pad or any other type of object or article that can be placed on the sleeping surface of the bed. Pillow 260 can be located in or comprise a climate zone, yet need not be in a particular location on the bed; rather, it may be located at any area of the bed that provides some sort of thermodynamic interaction with the sleeper. For example, pillow 260 may be placed next to the sleeper, abutting the sleeper's sides and/or arm.

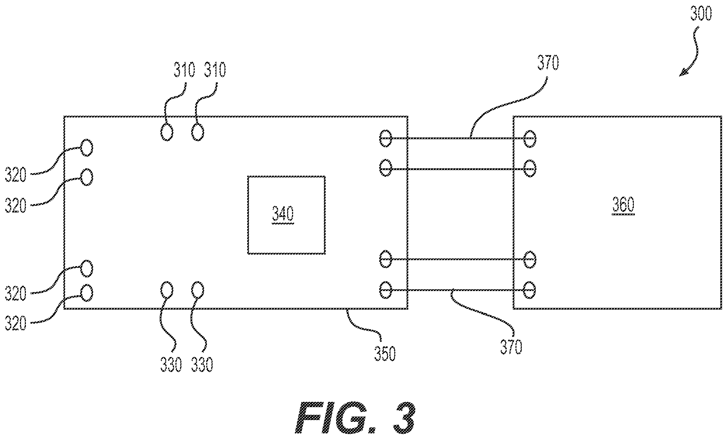

The various sensors described above can be connected directly or wirelessly to a control system. Fig. 3 discloses control system 300, which can include controller 350 and thermoelectric controller 360. In an example embodiment, control system 300 is responsible for receiving inputs from all available sensors, determining which zones to heat/cool, and instructing the thermoelectric energy source to perform particular heating and/or cooling actions. In the example embodiment of Fig. 3, controller 350 is configured to receive power inputs 310, temperature inputs 320, and non-temperature physiological inputs 330. Power inputs 310 may include, for example, a connector that plugs into a power outlet near the bed. Temperature inputs 320 include any temperature sensors on glabrous and/or non-glabrous skin of the user. Non-temperature physiological inputs 330 may include measurements of heart rate, blood pressure, oxygen levels, and so on. Other physiological inputs may be incorporated into the design as well. The particular number of input ports shown in Fig. 3 is not intended to be limiting-any number of sensors may be used.

In an example embodiment, controller 350 receives various inputs and determines, via a logic processor 340, how to heat and/or cool various zones of bed 100. For example, logic processor 340 may be capable of determining sleep onset based on information gathered from the user using predetermined sleep factors. Predetermined sleep factors include any factor relevant to the user's sleep. Examples include the circadian cycle of temperature variation, the time of day or night, the user's temperature on glabrous or non-glabrous skin sites, the user's heart rate, blood pressure, or blood oxygen levels, and so on. Logic processor 340 can be equipped with data regarding the natural circadian cycle of temperature variation. Using that data and comparing it to the data measured from the user, logic processor 340 can determine the appropriate method of facilitating sleep for the user. After determining a method of facilitating sleep, logic processor 340 causes controller 350 to communicate instructions to thermoelectric controller 360 via an electrical interface 370. Thermoelectric controller 360 is capable of relaying instructions to the thermoelectric device itself, which provides heating or cooling as desired. The controller 350 may also be equipped with a data logging or recording function to retain information about a sleeper during sleep and may be recovered at a later time for analysis.

In another embodiment, control system 300 is capable of receiving inputs directly from the user. For example, if the user feels too cold to sleep, the user can indicate this to control system 300, which will take appropriate measures based on a predetermined programmed response. Control system 300 may combine the inputs from a user with other factors to determine the optimal method of heating and/or cooling the bed.

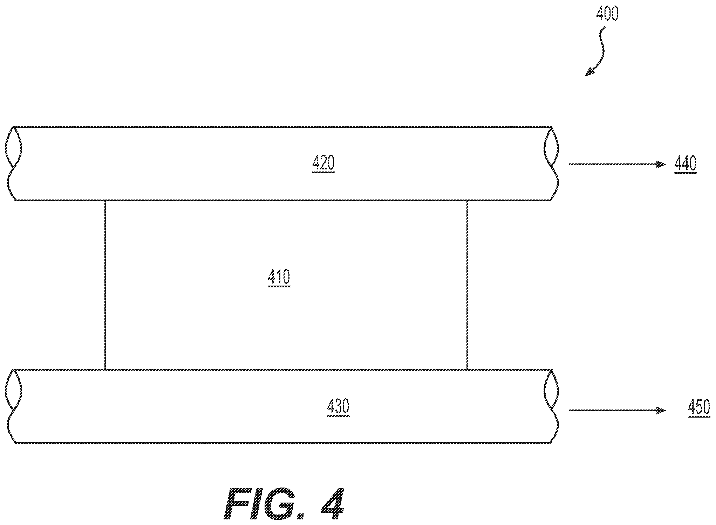

Fig. 4 discloses a thermoelectric device 400 for use in the example embodiments described above, as well as other embodiments. Thermoelectric device 400 is capable of providing warming and/or cooling capacity to different areas of the bed. In an example embodiment, thermoelectric device 400 includes an energy source 410. Energy source 410 may use, for example, electrical energy in order to heat or cool a fluid. While Fig. 4 shows energy source 410 as a single device that provides both heating and cooling services, it may alternatively comprise two devices that are responsible for heating and cooling, respectively. In one embodiment, energy source 410 uses heat created by cooling a fluid in conjunction with the cooled fluid to simultaneously warm and cool different locations of the bed.

In an example embodiment, energy source 410 can be operatively connected to pipes that carry heated or cooled fluid to various areas of the bed. In the embodiment of Fig. 4, a cold-side air duct 420 and a warm-side air duct 430 is depicted. The cold-side air duct 420 is attached to energy source 410 such that a cooling air flow 440 is directed through cold-side air duct 420. Cooling air flow 440 may be directed to multiple different zones of the bed depending on the needs of the user. The warm-side air duct 430 is attached to energy source 410 such that a warming air flow 450 is directed through warm-side air duct 430. Warming air flow 450 may be directed to multiple different zones of the bed depending on the needs of the user. Cooling air flow 440 and warming air flow 450 may be sent to different areas of the bed simultaneously.

While specific embodiments have been described in detail in the foregoing detailed description and illustrated in the accompanying drawings, it will be appreciated by those skilled in the art that various modifications and alternatives to those details could be developed in light of the overall teachings of the disclosure and the broad inventive concepts thereof. It is understood, therefore, that the scope of the present disclosure is not limited to the particular examples and implementations disclosed herein, but is intended to cover modifications within the scope thereof as defined by the appended claims and any and all equivalents thereof.

## Claims

1. A climate-controlled bed (100), comprising:
a first thermal energy source, wherein the first thermal energy source is capable of simultaneously providing cooling and warming to separate air streams directed to different areas of the bed sleeping surface; and
a control system that regulates a temperature of the bed via the first thermal energy source,
wherein the control system varies the temperature during a sleep cycle of the user based on at least one predetermined sleep factor.

2. The bed of claim 1, further comprising a sensor (220) configured to monitor a physiological temperature of a user, wherein the control system utilizes data from the sensor.

3. The bed of claims 1 or 2, wherein the control system is a closed-loop feedback system.

4. The bed of any of claims 1-3, wherein the sleep factors comprise at least one of the natural circadian cycle of temperature variation, the local time, and the user's current temperature.

5. The bed of any of claims 1-4, further comprising a second sensor configured to monitor at least a heart rate, physical movement, sweating, blood pressure, or a thermal state of the user.

6. The bed of any of claims 1-5, further comprising a cooling source configured to decrease the temperature of at least a portion of the bed.

7. The bed of any of claims 1-6, further comprising a second thermal energy source disposed on the bed.

8. The bed of any of claims 1-7, wherein the control system varies the temperature via heating at least a portion of an article disposed on the bed.

9. The bed of claim 8, wherein the article is one of a pillow and a heating pad.

10. A method of controlling temperatures of a bed (100), comprising
providing an energy source, wherein the energy source is capable of simultaneously
providing cooling and warming to separate air streams directed to different areas of the bed sleeping surface;
monitoring temperature data of a user via a control system;
modifying the temperature of the bed, via the energy source, based on the user's temperature data compared to a target temperature customized to the user.

11. The method of claim 10, wherein monitoring the temperature of the user comprises placing sensors on at least one glabrous skin site and optionally on at least one non-glabrous skin site.

12. The method of claims 10 or 11, further comprising monitoring at least one of heart rate, physical movement, and sweating of the user.

13. The method of any of claims 10-12, wherein the ideal temperature customized to the user is based on data representing a natural circadian cycle of temperature variation.

14. The method of any of claims 10-13, wherein the ideal temperature customized to the user is based on a request by the user.

15. The method of any of claims 10-14, wherein the energy source is provided on the bed.

## Patentansprüche

1. Klimagesteuertes Bett (100), umfassend:
eine erste thermische Energiequelle, wobei die erste thermische Energiequelle in der Lage ist, gleichzeitig verschiedenen Luftströmen, die auf verschiedene Bereiche der Schlafoberfläche des Bettes gerichtet sind, eine Kühlung und Erwärmung bereitzustellen; und ein Steuerungssystem, das eine Temperatur des Bettes mittels der ersten thermischen Energiequelle steuert, wobei das Steuerungssystem die Temperatur während eines Schlafzyklus des Benutzers basierend auf mindestens einem vorherbestimmten Schlaffaktor variiert.

2. Bett nach Anspruch 1, ferner umfassend einen Sensor (220), der zur Überwachung einer physiologischen Temperatur eines Benutzers konfiguriert ist, wobei das Steuerungssystem Daten vom Sensor verwendet.

3. Bett nach Anspruch 1 oder 2, wobei das Steuerungssystem ein Feedbacksystem mit geschlossenem Regelkreis ist.

4. Bett nach einem der Ansprüche 1 bis 3, wobei die Schlaffaktoren mindestens eines aus einem natürlichen Schlaf-Wach-Rhythmus von Temperaturschwankungen, der Ortszeit und der gegenwärtigen Temperatur des Benutzers umfasst.

5. Bett nach einem der Ansprüche 1 bis 4, ferner umfassend einen zweiten Sensor, der zur Überwachung mindestens eines aus einer Herzfrequenz, physischer Bewegung, Schwitzen, Blutdruck, Wärmezustand des Benutzers ist.

6. Bett nach einem der Ansprüche 1 bis 5, ferner umfassend eine Kühlquelle, die zur Senkung der Temperatur mindestens eines Teils des Bettes konfiguriert ist.

7. Bett nach einem der Ansprüche 1 bis 6, ferner umfassend eine zweite thermische Energiequelle, die auf dem Bett angeordnet ist.

8. Bett nach einem der Ansprüche 1 bis 7, wobei das Steuersystem die Temperatur über das Erwärmen mindestens eines Teils eines Artikels, der auf dem Bett angeordnet ist, variiert.

9. Bett nach Anspruch 8, wobei der Artikel eines aus einem Kopfkissen und einem Heizpad gebildet ist.

10. Verfahren zur Steuerung von Temperaturen eines Bettes (100), umfassend Bereitstellen einer Energiequelle, wobei die Energiequelle in der Lage ist, gleichzeitig verschiedenen Luftströmen, die auf unterschiedliche Bereiche der Schlafoberfläche des Bettes gerichtet sind, eine Kühlung und eine Erwärmung bereitzustellen; Überwachen von Temperaturdaten eines Benutzers über ein Kontrollsystem; Modifizieren der Temperatur des Bettes, über die Energiequelle, basierend auf den Temperaturdaten des Benutzers im Vergleich zu einer Zieltemperatur, die an den Benutzer angepasst wurde.

11. Verfahren nach Anspruch 10, wobei das Überwachen der Temperatur des Benutzers das Anordnen von Sensoren auf mindestens einer haarlosen Hautstelle und optional aus mindestens einer nicht-haarlosen-Hautstelle umfasst.

12. Verfahren nach Anspruch 10 oder 11, ferner umfassend das Überwachen mindestens eines aus einer Herzfrequenz, physikalischer Bewegung, und Schwitzen des Benutzers.

13. Verfahren nach einem der Ansprüche 10 bis 12, wobei die Idealtemperatur, die an den Benutzer angepasst wurde, auf Daten basiert, die einen natürlichen Schlaf-Wach-Rhythmus von Temperaturschwankungen darstellen.

14. Verfahren nach einem der Ansprüche 10 bis 13, wobei die Idealtemperatur, die auf den Benutzer zugeschnitten wurde, auf der Anfrage durch den Benutzer basiert.

15. Verfahren nach einem der Ansprüche 10 bis 14, wobei die Energiequelle auf dem Bett bereitgestellt wird.

## Revendications

1. Lit à atmosphère régulée (100), comprenant:
une première source d'énergie thermique, dans lequel la première source d'énergie thermique est en mesure de fournir simultanément du froid et du chaud pour séparer des courants d'air dirigés vers différentes zones de la surface de sommeil de lit ; et
un système de commande qui régule une température du lit via la première source d'énergie thermique, dans lequel le système de commande fait varier la température pendant un cycle de sommeil de l'utilisateur sur la base d'au moins un facteur de sommeil prédéterminé.

2. Lit selon la revendication 1, comprenant en outre un capteur (220) configuré pour surveiller une température physiologique d'un utilisateur, dans lequel le système de commande utilise des données provenant du capteur.

3. Lit selon les revendications 1 ou 2, dans lequel le système de commande est un système de rétroaction à boucle fermée.

4. Lit selon l'une quelconque des revendications 1 à 3, dans lequel les facteurs de sommeil comprennent au moins l'un parmi un cycle circadien naturel de variation de température, l'heure locale, et la température actuelle d'utilisateur.

5. Lit selon l'une quelconque des revendications 1 à 4, comprenant en outre un second capteur configuré pour surveiller au moins un rythme cardiaque, le mouvement physique, la transpiration, la pression sanguine, ou un état thermique de l'utilisateur.

6. Lit selon l'une quelconque des revendications 1 à 5, comprenant en outre une source de refroidissement configurée pour diminuer la température d'au moins une partie du lit.

7. Lit selon l'une quelconque des revendications 1 à 6, comprenant en outre une seconde source d'énergie thermique disposée sur le lit.

8. Lit selon l'une quelconque des revendications 1 à 7, dans lequel le système de commande fait varier la température via le chauffage d'au moins une partie d'un article disposé sur le lit.

9. Lit selon la revendication 8, dans lequel l'article est l'un parmi un oreiller et un coussin chauffant.

10. Procédé de régulation de températures d'un lit (100), comprenant les étapes consistant à fournir une source d'énergie, dans lequel la source d'énergie est en mesure de fournir simultanément du froid et du chaud pour séparer des courants d'air dirigés vers différentes zones de la surface de sommeil de lit ; surveiller des données de température d'un utilisateur via un système de commande ; modifier la température du lit, via la source d'énergie, sur la base des données de température d'utilisateur comparées à une température cible personnalisée pour l'utilisateur.

11. Procédé selon la revendication 10, dans lequel la surveillance de la température de l'utilisateur comprend le fait de placer des capteurs sur au moins un site de peau glabre et facultativement sur au moins un site de peau non glabre.

12. Procédé selon les revendications 10 ou 11, comprenant en outre la surveillance d'au moins l'un parmi un rythme cardiaque, le mouvement physique, et la transpiration de l'utilisateur.

13. Procédé selon l'une quelconque des revendications 10 à 12, dans lequel la température idéale personnalisée pour l'utilisateur est basée sur des données représentant un cycle circadien naturel de variation de température.

14. Procédé selon l'une quelconque des revendications 10 à 13, dans lequel la température idéale personnalisée pour l'utilisateur est basée sur une demande de l'utilisateur.

15. Procédé selon l'une quelconque des revendications 10 à 14, dans lequel la source d'énergie est prévue sur le lit.
